# EUROPEAN PATENT APPLICATION

(11) **EP 1 230 909 A1**
(43) Date of publication of application: **14.08.2002**
(21) Application number: 01200464.4
(22) Date of filing: 09.02.2001
(51) Int. Cl.: A61K 7/42

(54) **Sun protection product**

(71) Applicant: JOHNSON & JOHNSON GmbH, 40474 Düsseldorf (DE)
(72) Inventor: Von Stetten, Otto, 52072 Aachen (DE); Martens, Nicolas, 53604 Bad Honnef (DE); Langhals, Maria, 53229 Bonn (DE); Lange, Rainer, 53604 Bad Honnef (DE); Johnston, Andrew, Denham Uxbridge Middlesex UB9 4EQ (GB); Lindermeier, Tanja, 53111 Bonn (DE)
(74) Representative: Wante, Dirk

(57) **Abstract**

A sun protection product comprising a carrier to which has been applied a sunscreen composition.

## Description

### Background of the Invention

This invention concerns sun protection products comprising a carrier material to which a suitable sun protective composition has been applied. In particular the invention relates to a wipe to which is applied a sunscreen lotion. The invention further concerns the manufacture and use of such products.

Public awareness of the risks related to the exposure of the skin to sunlight today is higher than ever before and in particular the harm caused by the ultraviolet spectrum of sunrays has become well recognized. One of the more important factors that contributed thereto is the increased incidence of skin conditions such as actinic keratosis and carcinomas such as malignant melanoma in many countries during the last decades.

Obviously, there are several options to protect oneself from the harm caused by exposure, in particular by intense exposure, of the skin to sunlight. The simplest method is to avoid direct exposure of the skin to solar irradiation. For many people this is not desirable or feasible for a number of reasons. Therefore a plurality of cosmetic sunscreen products has been developed.

Initially these products contained chemical substances that absorb mainly UV-B rays, but with the recognition that sunlight in the UV-A region causes adverse effects on the human skin too, newer products containing filters against both UV-A and UV-B have been developed.

Sunscreen products have become widely spread in the market place and are available in a wide variety of forms such as lotions, oils, creams and the like, as well as with different sun protection factors (SPF). During the last couple of years the focus has been on the development of sunscreen products with still higher protective factors. Independently therefrom there remains room for further improvement as regards ease and comfort of application of sunscreen products.

State of the art sunscreen products comprise either only organic filters, or purely inorganic pigments, or a combination of both. To achieve the currently desired high protective factors fairly high amounts of these filters have to be incorporated in the formulations. To develop waterproof products special ingredients have to be added leading to fairly viscous formulations that cannot easily be spread and often cause a sticky feeling on the skin. A recent development has been the introduction of sunscreen sprays where, because of the low viscosity of the spraying liquid, an even distribution of the product on the skin is hardly possible.

Another problem associated with the sunscreen products currently available is that the protective effect appears only after a certain period of time, in particular after 15 and in most case after 20 minutes. Consumers often have the perception that upon usage of the sunscreen product the protection is instantly available which is not the case.

Therefore it is an object of the present invention to provide a sunscreen product which on the one hand offers sufficient protection against harmful solar irradiation, and on the other facilitates the application in particular the even distribution of the sun-protecting agents on the skin. This is especially important when used with small children.
It is a further object of this invention to provide a sun care product that provides instant protection, within a few minutes or at once.

It is still a further object to provide products of a size that it is sufficiently compact and that can be easily taken along and transported.

The sunscreen products of this invention not only meet these requirements but also show a number of other advantages that are outlined hereinafter.

### Summary of the Invention

The present invention concerns sun protection products comprising an appropriate solid carrier material to which a sun protective or sunscreen composition has been applied.

In particular the carrier material is an absorbent sheet, more in particular a wipe, and the sunscreen composition is a liquid, in particular a lotion. Preferred are wipes that have been sprayed or impregnated with the sunscreen composition.

In a further aspect, the invention provides a method of manufacturing a sunscreen product as specified herein comprising applying a sunscreen composition to the carrier. In particular there is provided a method of applying a sunscreen lotion to a wipe.

In still a further aspect the invention concerns the use of a sunscreen product as specified herein, in particular the use of a wipe, to which a sunscreen composition has been applied, to apply an effective amount of a sun protection composition to the skin. In a particular aspect the invention concerns the use of a sunscreen wipe to apply an effective amount of a sunscreen lotion to the skin.

Alternatively, the invention concerns a method of protecting the skin against adverse effects of solar irradiation, said method comprising applying a sun protection product as specified herein to the skin. In a particular aspect there is provided a method of protecting the skin against the adverse effects of solar irradiation, said method comprising applying a sunscreen wipe as defined herein to the skin.

### Detailed Description of the Invention

One embodiment of this invention is a sunscreen product that comprises a carrier material, in particular in the form of a sheet of absorbent or porous material. The carrier material can take the form of a tissue, a wipe, towel, towelette, and the like.

Carrier materials that can be used can be mono or multi-layered, woven or non-woven. They can be made of one or of several materials. Particularly preferred are non-woven materials that have a web structure of fibrous or filamentous nature, in which the fibres or filaments are distributed randomly or with a certain degree of orientation, the former being obtainable by air-laying or certain wet-laying processes, the latter in other wet-laying or in carding processes. The fibres or filaments can be natural, for example wood pulp, wool cotton, linen and the like, or synthetic, for example polyvinyls, polyesters, polyolefins, polyamides and the like.

Typically they have a weight per square meter in the range of 10 to 80 g/m², in particular of 20 to 70 g/m². Particular materials are of the non-woven type. Based on the raw material that has been used, two different types of products can be distinguished.

A first type of carriers is paper based. The raw materials for these carriers are made almost exclusively of cellulose-based fibres or filaments from plant cellular sources (pulp). These can be available from fresh wood-shavings or from recycled material (recycled paper). In a number of wipe applications, such as baby wipes, wipes for cleansing, wet paper towels and the like, high wet strength or firmness of the non-woven web is a desirable attribute. This can be achieved by the addition of binding materials. Examples of such materials are the so-called wet strength resins. In some cases additives are added in order to increase the softness of the end product.
In a second type use the web is made mainly of staple, e.g. based on cotton, wool, linen and the like.

Commercial products are made of cellulose fibres, synthetic fibres or mixtures of both. Polyester and polypropylene are known as suitable polymers for the preparation of synthetic fibres. Also in these products binders can be used to increase the firmness of the non-woven fabric.

Webs of increased strength can be obtained by using the so-called spunlace or hydro-entanglement technique. In this technique the individual fibres are twisted together so that an acceptable strength or firmness is obtained without using binding materials. The advantage of the latter technique is the excellent softness of the non-woven material.

Non woven materials that are made of a mixture of pulp and staple are alsoknown. Such materials are available with binding materials, in particular those mentioned above, or without binding materials. In the latter instance the non-woven is preferably made by the spunlace or hydro-entaglement procedure.

In a preferred embodiment of the present invention, the carrier material is made of cellulose pulp with a small amount of binding material. The amount of binder in the carrier material is in the range of 5 to 20 % (w/w).
In a particularly preferred embodiment the non-woven fabric is prepared by the water entanglement procedure and does not contain binding material.

The absorbing ability of the carrier material is of particular interest with regard to the applications envisaged by the present invention. During production the impregnating solution should be taken up quickly by the carrier. In certain embodiments of this invention the wipes will be packed in a stack of a plurality of wipes. In this instance the absorbing ability of the non-woven fabric should be such that a chromatographic effect (sinking down of the lotion) in the stack is avoided during storage. On the other hand it should be guaranteed that during the usage of the wipe the impregnating solution is delivered evenly to the skin and the active ingredients, i.e. the sunscreen filters are released quantitatively.

The absorbing capacity of the carrier material is determined essentially by three different parameters: the surface weight of the carrier material, the nature of the raw material used in the manufacture and the manufacturing process used.

For the applications according to the invention the carrier materials typically have a surface weight from 10 g/m2 to 80 g/m2, preferably from 30 to 70 g/m2 and more preferably from 40 to 60 g/m2. The selection of the raw material of which the non-woven carrier is made depends on the manufacturing procedure. Typically in the manufacture of non-woven carriers by the hydro-entanglement process, use is made of mixtures of cellulose fibres and synthetic fibres. The relative quantity of synthetic fibres in the non-woven fabric is from 0 to 100 % and preferably is between 10 and 70 %, more preferably in the range of 30 to 50% (all percentages being w/w).

The sunscreen compositions for use in the products of the invention can take a variety of forms. The compositions will preferably be liquid. Particular liquid compositions are lotions that can either be of the oil-in-water or water-in-oil type, the oil-in-water type being preferred.

Preferred lotions are those based on emulsions prepared by the so-called phase inversion technique. The phase inversion technique is described in more detail by F. Förster, F. Schambil, and H. Tesmann in Int. J. Cos. Sci. 1990 (12) 217.

According to this technique, oil-in-water formulations made with non-ionic emulsifiers typically undergo a phase inversion upon heating which means that within a particular temperature interval a change of the emulsion type takes place, i.e. from an oil-in-water to a water-in-oil emulsion. In this process the external continuous phase changes from being aqueous to an oily phase resulting in a drop of the electrical conductivity to virtually zero. The average temperature between that of maximal and of minimal conductivity is referred to as the phase inversion temperature ('PIT').

After heating to a temperature above the PIT, the emulsion is cooled below the PIT whereupon the inverse phase transfer takes place, i.e. from water-in-oil to oil-in-water. The resulting emulsions are usually referred to as 'PIT emulsions'.

The droplet size of the PIT emulsion depends on a number of factors. PIT emulsions with small droplet size can be obtained with emulsions forming microemulsions having a low surface tension between the oil and water phases at the phase inversion, or that form a laminar liquid crystalline phase.

Preferred are PIT emulsions that are finely dispersed, i.e. having a small droplet size and have low viscosity.

The oily phase in PIT emulsions comprises natural oils or natural oil derivatives, in particular of vegetal origin. Examples are linseed oil, palm oil, olive oil, castor oil, rapeseed oil, soja oil, and in particular peanut oil, coconut oil, sunflower oil and turnip seed oil. The oily phase may further comprise fatty components isolated from these natural oils, i.e. pure triglycerides or mixtures thereof, or the latter components having been prepared chemically. These so-called trigycerides are esters of glycerine with fatty acids or fatty acid mixtures. Preferred triglycerides are those glycerine esters derived from fatty acids, either saturated or unsaturated, having from 10 to 24, particularly from 14 to 20, preferably from 16 to 18 carbon atoms, for example palmitic, heptadecanoic, oleic or stearic acid, or mixtures thereof. Particularly preferred is glyceryl stearate, also referred to as stearin.

The oily phase may further comprise alkyl esters of fatty acids, wherein the alkyl group has from 1 to 4 carbon atoms. Preferred are C₁₋₄ alkyl esters of C₁₆₋₁₈ fatty acids, for example of palmitic, heptadecanoic, or stearic acid, in particular the methyl or ethyl esters, including mixtures thereof.

Of particular interest are oily phases that comprise a vegetable oil or a triglyceride in combination with an alkyl ester of a fatty acid.

The PIT emulsion further contains a non-ionic emulsifier.
Suitable non-ionic emulsifiers comprise:
polyethoxylated or propoxylated fatty alcohols, fatty acids or C₈₋₁₅ alkylphenols, having 2 to 30 ethoxy units and 0 to 5 propoxy units, or 1 to 5 propoxy units, prepared by reacting the starting alcohols with ethylene or propylene oxide;
mono- or diesters of polyethoxylated glycerine that with saturated or unsaturated C₁₂₋₁₈ fatty acids, having 1 to 30 ethoxy units;
glycerin mono- or diesters and sorbitan mono- or diesters of saturated or unsaturated fatty acids as well as ethoxylated derivatives thereof, the latter in particular having from 1 to 30 ethoxy units; C₈₋₂₂ alkyl mono- or oligoglycosides as well as ethoxylated derivatives thereof, the latter in particular having from 1 to 30 ethoxy units;
ethoxylated castor oil or hydrogenated castor oil, in particular having from 1 to 30 ethoxy units;
polyol fatty acid esters and in particular polyglycerine fatty acid esters, more in particular ricinoelic acid or hydroxy stearic acid esters; for example polyglycerine poly ricinoleic acid or polyglycerine poly 12-hydroxystearate; and mixtures thereof;
glycerine, polyglycerine, mono- and di-pentaerythrite, sugar derived alcohols such as sorbitol, alkylglucosides and polyglucosides, partially esterified with one ore more fatty acids or fatty acid mixtures;
trialkylphosphates as well as polyethoxylated derivatives thereof, the latter in particular having from 1 to 30 ethoxy units;
wool wax alcohols;
polysiloxane-polyalkyl-polyether copolymers and derivatives thereof;
mixed ethers of pentaerythrite, fatty acids, citric acid and fatty alcohols polyalkylene glycols;
glycerine carbonate.

As used herein the term fatty acid refers to saturated or unsaturated, straight or branch chained alkanoic acids, optionally substituted with one or more hydroxy groups.

Particular useful emulsifiers comprise an emulsifier system containing a mixture of a hydrophilic and hydrophobic emulsifier.
Hydrophilic emulsifiers comprise ethoxylated fatty alcohols or fatty acids. Examples of the former are ethoxylated C₁₆₋₂₂ alcohols such as for example cetyl, palmoleyl, stearyl, isostearyl and oleyl alcohol and mixtures thereof wherein the number of ethoxyl groups per molecule is in the range of 1 to 35, preferably from 1 to 20, more preferably from 10 to 20.
Examples of ethoxylated fatty acids are ethoxylated C₁₂₋₂₂ alkylcarbonic acids such as, for example, palmitinic, palmoleinic, steraic, isosearic acid and mixtures thereof, wherein the number of ethoxy groups is in the range of 5 to 50, in particular from 15 to 35.
Hydrophobic emulsifiers comprise polyethoxylated glycerin fatty acid mono- and diesters having 1 to 30 ethoxy units, i.e. polethoxylated glycerin wherein between 1 and 2 of the hydroxy functions have been esterified with 1 or 2 fatty acids or fatty acid mixtures.
The w/w ratio of the hydrophilic emulsifier components to the hydrophobic emulsifier components is in the range of 10 : 90 to 90 : 10, in particular 25 :75 to 75:25, more in particular in the range of 40 : 60 to 60 : 40.
The PIT emulsions for use in the products according to the invention in particular contain from 20 to 90 %, more in particular from 30 to 80 % and preferably 30 to 60 % of water. The remainder making up the formulation comprises the oily phase, the emulsifiers and other components. The oily phase typically comprises from 10 to 80 %, in particular from 40 to 70 % of the formulation. Preferred are emulsion wherein the w/w ratio of the oil and aqueous phases are about 1:1. The emulsifiers are present in an amount that is in the range of 1 to 25 %, in particular 5 to 20 % and more in particular 5 to 15 %.
The phase inversion temperature typically is in the range of 20 to 95 °C, in particular in the range of 40 to 95 °C.
The PIT lotions for use in the present invention will contain one or more light absorbing or light reflecting substances, in particular those mentioned herein. These can be hydrophilic or hydrophobic. In the former instance these substances will be solved into the aqueous phase while in the latter into the oily phase.
Particular PIT emulsions that can be used in the sunscreen compositions of this invention are described for example in WO-00/51427 and in WO-00/71676

The sunscreen compositions of the oil in water type are prepared by the phase inversion technique preferably have a viscosity of equal to or below 200 mPas, more preferably equal to or below 150 mPas, most preferably equal to or below 100 mPas. The particle size of the oil droplets is in the range of 50 to 300 nm, in particular in the range of 50 to 200 nm, and preferably is 100 nm or smaller. These compositions are particularly attractive in that they show good spreading and impregnating properties.

One of the problems using a carrier material to deliver sunscreens to the skin is the quantitative release of the active material to the skin. Typically, actives are absorbed to the carrier and inadequately released.

Quite unexpectedly it has been found that when using these compositions, the sunscreen agents are not strongly absorbed to the carrier material, but are completely or almost completely transferred to the skin.

This is demonstrated by the fact that the concentration of sunscreen substances in the liquid that is delivered by a wipe is higher than that in the liquid that was used to impregnate the wipe.

Sunscreen filters that can be used in the products according to this invention are, for example, organic substances that are liquid or solid at room temperature. These filters are capable of absorbing the ultraviolet spectrum from sunlight and to emit it as radiation of longer wavelengths, in particular infrared radiation, i.e. warmth. The filters can be water-soluble as well as oil soluble.

The following are a number of oil-soluble sunscreen substances:
- 3-benzylidene camphor or 3-benzylidene norcamphor, or derivatives of these substances, for example 3-(-4methylbenzylidene)-camphor;
- derivatives of 4-aminobenzoic acid, for example 4-(dimethylamino)-benzoic acid-2-ethylhexyl ester, 4-(dimethylamino)-benzoic acid-2-octyl ester and 4-(dimethylamino)-benzoic acid amyl ester;
- esters of cinnamic acid, for example 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (octocrylene);
- esters of salicylic acid, for example salicylic acid-2-ethylhexyl ester, salicylic acid-4-isopropyl benzylester, salicylic acid-homomethylesters;
- derivatives of benzophenone, for example 2-hydroxy-4-methoxybenzophenone, 2-hydroxy-4-methoxy-4'-methylbenzophenone, 2,2'-dihydroxy-4-methoxybenzophenone;
- esters of benzalmalonic acid, for example 4-methoxybenzalmalonic acid di-2-ethylhexyl ester;
- triazine derivatives such as for example 2,4,6-trianilino-(p-carbo-2'-ethyl-1'-hexyloxy)-1,3,5-triazine and octyl triazones as described in EP-A1-0818450;
- propane-1,3-diones such as for example 1-(4-tert.butylphenyl)-3-(4'-methoxyphenyl)-propane-1,3-dione;
- ketotricyclo(5.2.1.0)decane derivatives such as those described in EP-B1-0694521

Water soluble sunscreen agents that can be used in the products of the invention are for example:
- 2-phenylbenzimidazole-5-sulfonic acid and the base-addition salts thereof;
- sulfonic acid derivatives of benzophenones, for example 2-hydroxy-4-methoxybenzophenone-5-sulfonic acid and the base-addition salts thereof;
- sulfonic acid derivatives of 3-benzylidenecamphor such as for example 4-(2-oxo-bornylidenemethyl)-benzolsulfonic acid and the base-addition salts thereof.

Suitable base-addition salts of the above mentioned water soluble sunscreen agents are for example alkali metal, alkaline earth metal, ammonium, mono-, di-, trialkylammonium, mono-, di-, trialkanolammonium, glucammonium salts, and lysinates or arginates.

Typical UVA light absorbing filters are the derivatives of benzoyl methane such as for example 1-(4'-tert.butylphenyl)-propane-1,3-dione, 4-tert.butyl-4'-methoxydibenzoyl methane (Parsol 1789) or 1-phenyl-3-(4'-isopropylphenyl)-propane-1,3-diones.

The sunscreen composition can evidently also contain a mixture of an effective amount of one or more UV-A and UV-B filters.

Apart from the above mentioned light protection filters, finely divided light-absorbing, light-diffusing or light reflecting pigments can also be used as light-blocking substances. These substances can be metal oxides or metal salts. Compounds that are useful in this application in particular are titanium oxide, zinc oxide, iron oxide, aluminium oxide, cerium oxide, zirconium oxide, silicates (talcum), barium sulphate and zinc stearate. The particle size of such substances typically is smaller than 100 nm. In a preferred embodiment the particle size is in the range of 5 to 50 nm and in a particularly preferred embodiment in the range 15 and 30 nm. The particles can be spherical although particles having other shapes can also be used.

Other possible UV filters are those mentioned in P. Finkel's publication in SOFW-Journal 122, 543 (1996).

Apart from both groups of primary light protecting filters that are mentioned above there can also be used secondary light protecting factors. These pertain to the class of anti-oxidants and their activity is based on the interruption or decrease of the photochemical processes caused by solar radiation upon penetration in the skin.

Typical examples of secondary light protecting agents are amino acids such as for example glycine, histidine, tyrosine and tryptophane. Moreover, derivatives of these amino acids are frequently used, such derivatives being for example D,L-carnosine, D-carnosine, L-carnosine and derivatives of these compounds. Further agents that can be used are carotinoides, carotenes (for example α-Carotene, β-Carotene and lycopene) and derivatives thereof, , lipoic acid and derivatives thereof, aurothioglucose, propylthiouracil and other thioles (for example thioredoxine, glutathione, cysteine, cystamine and glycosyl, N-acetyl, methyl, ethyl, propyl, amyl, butyl and lauryl, palmitoyl, oleyl, γ-linoleyl, cholesteryl and glyceryl esters) and their salts. Further examples are dilauryl thiodipropionates, distearyl thiopropionates, thiodipropionic acids and derivatives thereof (for example esters, ethers, peptides, lipids, nucleotides, nucleosides and salts), sulfoximine compounds (for example butionin sulfoximin, homocystein sulfoximin, butionin Sulfon, penta- hexa-, and heptathione sulfoximin).

Chelating agents can also be used in small concentrations (for example α-hydroxy fatty acids, palmeate acid, phytic acid, lactoferrin), α-hydroxyacids (for example citric acid, lactic acid , malic acid), humic acid, bile acid, bile extracts, bilirubin, biliverdin, EDTA, EGTA and derivatives thereof;
unsaturated fatty acids and derivatives thereof such as for example -linolenic acid, linoleic acid, oleic acid; folic acid and derivatives thereof,
ubiquinones and ubiquinol and derivatives thereof, vitamin C and derivatives thereof, tocopherol and derivatives thereof (for example vitamin E acetate), Vitamin A and derivatives thereof, coniferyl benzoates of benzoinic acid, rutinic acid and derivatives thereof, α-Gylcosylrutin, Ferula acid, furfurylidene glucital, carnosin, butyl hydroxytoluene, hydroxyanisol, nordihydroguaiac resin acid, nordihydroguaiaret acid, trihydroxybutyrophenone, ureic acid and derivatives thereof, mannose and derivatives thereof, superoxide-dismutase, zinc and derivatives thereof such as zinc oxide etc., selenium and derivatives thereof; stilbene and derivatives thereof and derivatives of these active substances that may be used when executing this invention.

Typically the sunscreen compositions contain further skin caring and/or active ingredients like emollients, oils, plant extracts, vitamins, etc. Oils can be of natural or synthetic origin, e.g. vegetable oils or mineral oils or the group of silicones.

The group of emollients comprises lipids like lanolin , lanolin alcohols, lanolin acids, polyethoxylated or acylated lanolin or lanolin derivatives, lecithin and lecithin derivatives, fatty alcohols , either linear or branched with chain lengths between C6 and C40, and their esters with organic acids, e.g. carbonic acids or polyacids containing between 2 and 30 C atoms, branched, aromatic or linear including hydroxy or amino acids, fatty acids and fatty acid esters with alcohols or poly alcohols containing between 2 and 40 C atoms, branched, aromatic or linear, sterols found in the unsaponifiable fraction of e.g. avocado oil, almond oil, soybean oil, etc. like soy phytosterol, β-sitosterol, β-sitosteryl laurate, β-sitosteryl stearate, etc. natural and synthetic waxes, e.g. bees wax, purcelline, shea butter, cocoa butter, ceresin, ozokerit, vaseline,micro wax, carnauba wax candelilla wax and alike, substituted cyclohexanes like di-n-octylcyclohexane, Guerbet carbonates, e.g. bis-2-octyl dodecylcarbonate, dialkyl ethers like di-n-octyl ether, etc.

Examples of oils are natural oils, e.g. almond oil, soybean oil, wheat germ oil, avocado oil, jojoba oil, linseed oil, sesame oil, walnut oil, sunflower oil, olive oil, etc., mineral and paraffin oil and synthetic oils comprising mono-, di-, triglycerides as well as mixtures thereof, etc.

Silicones comprise e.g. dimethyl polysiloxanes, methylphenyl polysiloxanes, cyclic silicones and silicon compounds derivatised with amino-, fatty acid, alcohol-, polyether, epoxy-, and/or alkyl groups.

The compositions may also contain film-forming substances like chitosan and derivatives thereof, derivatives of poly acrylic acid, polyvinyl pyrrolidone and its derivatives, etc.

The compositions may further contain active ingredients such as anti-microbials, anti inflammatory agents, anti irritating compounds, moisturising agents, plant extracts, vitamines, and the like. Examples of such ingredients comprise complexes of PVP and hydrogen peroxide, diclofenac, acetyl salicylic acid, salicylates, ibuprofen, bisabolol, mimosa extract (mimosa tenuiflora), hyaluronic acid, propylene glycol, glycerin, chondroitin sulfate, plant extracts, bisabolol, panthenol, tocopherol, actives for anti-stinging, anti-irritants, anti-dandruffs, anti-ageing agents e.g. retinol, melibiose etc. Other suitable actives are e.g. medicago officinalis, actinidia chinensis, allantoin, aloe barbadensis, anona cherimolia, anthemis nobilis, arachis hypogaea, arnica montana, avena sativa, beta-carotene, bisabolol, borago officinalis, butylene glycol, calendula officinalis, camellia sinensis, camphor, candida bombicola, capryloyl glycine, carica papaya, centaurea cyanus, cetylpyridinium chloride, chamomilla recutita, chenopodium quinoa, chinchona succirubra, chondrus crispus, citrus aurantium dulcis, citrus grandis, citrus limonum, cocos nucifera, coffea arabica, crataegus monogina, cucumis melo, dichlorophenyl imidazoldioxolan, enteromorpha compressa, equisetum arvense, ethoxydiglycol, ethyl panthenol, farnesol, ferulic acid, fragaria chiloensis, gentiana lutea, ginkgo biloba, glycerin, glyceryl laurate, glycyrrhiza glabra, hamamelis virginiana, heliotropine, hydrogenated palm glycerides, citrate, hydrolyzed castor oil, hydrolyzed wheat protein, hypericum perforatum, iris florentina, juniperus communis, lactis proteinum, lactose, lawsonia inermis, linalool, linum usitatissimum, lysine, magnesium aspartate, magnifera indica, malva sylvestris, mannitol, mel, melaleuca alternifolia, mentha piperita, menthol, menthyl lactate, mimosa tenuiflora, nymphaea alba, olaflur, oryza sativa, panthenol, paraffinum liquidum, PEG-20M, PEG-26 jojoba acid, PEG-26 jojoba alcohol, PEG-35 castor oil, PEG-40 hydrogenated castor oil, PEG-60 hydrogenated castor oil, PEG-8 caprylic/capric acid, persea gratissima, petrolatum, potassium aspartate, potassium sorbate, propylene glycol, prunus amygdalus dulcis, prunus armeniaca, prunus persica, retinyl palmitate, ricinus communis, rosa canina, rosmarinus officinalis, rubus idaeus, salicylic acid, sambucus nigra, sarcosine, serenoa serrulata, simmondsia chinensis, sodium carboxymethyl betaglucan, sodium cocoyl amino acids, sodium hyaluronate, sodium palmitoyl proline, stearoxytrimethylsilane, stearyl alcohol, sulfurized TEA-ricinoleate, talc, thymus vulgaris, tilia cordata, tocopherol, tocopheryl acetate, trideceth-9, triticum vulgare, tyrosine, undecylenoyl glycine, urea, vaccinium myrtillus, valine, zinc oxide, zinc sulfate.

The sunscreen products according to the invention can be made by preparing the impregnating solution according to the PIT procedure and subsequently impregnating the carrier material.
When using certain UV filters, in particular the UVA filter butyl methoxybenzoylmethane, care must be taken to avoid contamination with metal ions, especially iron, since the solution would turn red.

Hence the invention concerns a process for preparing a sunscreen product as defined herein, said process comprising contacting a suitable carrier with a sunscreen composition. In particular said process comprises contacting a wipe with a sunscreen lotion, more in particular impregnating or spraying a wipe with a sunscreen lotion. The latter preferably is a PIT formulation as described herein.

In a particular execution, the carrier material is cut into strips the transversal size of which being similar to the size of the sheet, in particular the tissue or wipe. Subsequently the carrier strips are folded according to methods generally known and applied in the art. The thus folded strips are moistened with a sunscreen composition, in particular with a sunscreen composition, in particular a lotion as described herein, said moistening preferably comprising spraying or dripping. Or the strips can first be moistened and subsequently be folded.
The strips can also be impregnated with the sunscreen composition by immersing in or running the strip through a bath containing the composition.
In a further step, the strips are cut so that the desired size of the sheets, in particular of the wipes, is obtained. The thus obtained sheets (or wipes) can be packed individually or can be stacked in a determined number, e.g. a number between 10 and 30, preferably between 15 and 25, most preferably about 20, and the stack then packed in a suitable package, for example a plastic wrap, box and the like.

The sunscreen products according to this invention, in particular the wipes impregnated with a lotion containing sunscreen agents, can be packed individually for example in a pouch, and a number of pouches can be packed e.g. in a suitable card or plastic box. Or they can be stacked in the same way as wet wipes in a box. Each wipe contains a predetermined amount of sunscreen agent sufficient to treat a certain surface of skin.

The invention further concerns the use of a sunscreen product as defined above to protect the skin from deteriorating effects of sunrays. Or alternatively, the invention concerns a method of treating the skin with a sunscreen product according to the invention, said method comprising contacting the skin with the carrier holding the sunscreen composition, in particular contacting the skin with a wipe holding the sunscreen composition, more in particular rubbing the skin with a wipe being impregnated with a suitable amount of a sunscreen lotion.

The softness of the wipes is positively influenced when impregnated by a lotion prepared by the phase inversion technique.

The products of the invention facilitate the application of sunscreen agents to the skin in that the sunscreen composition, in particular the sunscreen lotion is quite easily distributed on the skin surface. It has further been found that when applied to the skin, the sunscreen composition is finely distributed and by the optimal distribution of sunscreen agent or agents on the skin the desired sunscreen protection factor is obtained immediately or almost immediately after application. This in contrast to the usual sunscreen products that need to be applied in advance, usually about 20 to 30 minutes, prior to the exposure of the skin to the sun. This is an important advantage in that there is a great tendency with users of sunscreen products to immediately expose the skin to the sun as it often turns out to be unpractical to hide the skin from the sun for about half an hour. This in particular is important for an adequate protection of small children, in particular of infants and babies.

### Examples

As used in the following examples, all percentages are by weight.

### Example 1: Formulation

| | |
|---|---|
| Water | 63.91% |
| | |
| Glyceryl stearate/ Ceteareth 20 ™ / Ceteareth 12™/ Cetearyl alcohol/ cetyl palmitate mixture ('Emulgade SE-PF' ™) | 7.30% |
| | |
| Ceteareth 20 ('Eumulgin B2' ™) | 4.70% |
| | |
| dicapryl ether | 4.00% |
| cetearyl isononanoate | 5.00% |
| Cocoglyceride | 2.00% |
| | |
| 4-Methylbenzylidene camphor | 3.00% |
| Octyl methoxycinnamate | 6.00% |
| Butyl methoxydibenzoylmethane | 2.00% |
| Tocopheryl acetate | 0.50% |
| | |
| Phenoxyethanol | 1.00% |
| PEG-4 laurate/ iodopropynyl butylcarbamate | 0.10% |
| Perfume | 0.15% |
| Tetrasodium EDTA | 0.20% |
| | |
| Cetylpyridium chloride | 0.10% |
| Citric acid | 0.04% |
| | |
| Ceteareth-20 ™ is ethoxylated cetostearyl alcohol having 20 ethoxy units. | |
| Ceteareth-12 ™ is ethoxylated cetostearyl alcohol having 12 ethoxy units. | |

The above ingredients are mixed and subsequently slowly warmed above the PIT temperature. This is determined by measuring the electrical conductivity of the mixture. Subsequently the mixture is allowed to cool slowly to room temperature. The thus obtained PIT formulation is has the appearance of a clear solution with low viscosity.

### Example 2: Sun Protection Wipe

Dry hydro-entangled carrier material made of fabric having a surface weight of 50 g/m2 was cut into strips. The strips were sprayed in the conventional manner with the PIT solution as prepared in example 1. Liquid addition was set at 6 g per wipe. Subsequently the strips were folded and cut.

## Claims

1. A sunscreen product comprising a solid carrier and a sunscreen composition.

2. A sunscreen product according to claim 1 wherein the carrier is an absorbent sheet and the sunscreen composition is a lotion.

3. A sunscreen product according to claim 2 wherein the carrier is a non-woven absorbent sheet and the sunscreen composition is a lotion of the oil in water type.

4. A sunscreen product according to claim 3 wherein the lotion of the oil-in-water type prepared by the phase inversion technique.

5. A sunscreen product according to claim 4 wherein the lotion comprises a fatty component which is a triglyceride and a non-ionic emusfier which comprises a polyethoxylated C₁₆₋₂₂ alcohol.

6. A sunscreen product according to claims 4 or 5 wherein the droplet size is equal to or below 100 nm and the viscosity is equal to or below 100 mPas.

7. A sunscreen product according to claims 1 to 6 wherein the sunscreen composition contains one or more UV A and/or UV B filters.

8. A sunscreen product according to any of claims 1 to 6 wherein the sunscreen composition contains light absorbing pigments.

9. A sunscreen product according to claims 3 to 8 wherein the non-woven material is cellulose based.

10. A sunscreen product according to claims 3 to 8 wherein the non-woven material comprises a mixture of cellulose and synthetic fibres and is manufactured by the spunlace procedure.

11. An oil in water emulsion prepared according to the phase inversion technique comprising a fatty component which is a triglyceride, a non-ionic emusfier which is a polyethoxylated C₁₆₋₂₂ alcohol, and a sunscreen agent.

12. An emulsion according to claim 10 wherein the droplet size is equal to or below 100 nm and the viscosity is equal to or below 100 mPas.

13. An emulsion according to claims 10 or 11 wherein the sunscreen agent comprises one or more UV A filters and/or one or more UV B filters.

14. An emulsion according to claims 10 or 11 wherein the sunscreen agent comprises light absorbing pigments.

15. A process for preparing a sunscreen product as claimed in claims 1 - 10 **characterized in that** a suitable carrier is contacted with a sunscreen composition.

16. A process according to claim 15 wherein the carrier is a wipe and the sunscreen composition is an oil-in-water lotion prepared according to the PIT technique, wherein the wipe is impregnated or sprayed with the said sunscreen lotion.
